# EUROPEAN PATENT SPECIFICATION

(11) **EP 2 295 154 B1**
(45) Date of publication and mention of the grant of the patent: **14.11.2012**
(21) Application number: 10176595.6
(22) Date of filing: 14.09.2010
(51) Int. Cl.: B06B 1/06

(54) **Ultrasonic transducer, ultrasonic probe and producing method**
Ultraschallwandler, Ultraschallsonde und Herstellungsverfahren
Transducteur ultrasonique, sonde ultrasonique et procédé de production

(30) Priority: 15.09.2009 JP 2009212663; 28.09.2009 JP 2009222007; 29.09.2009 JP 2009223691
(43) Date of publication of application: 16.03.2011
(60) Divisional application: 11179029.1
(73) Proprietor: Fujifilm Corporation, Minato-ku Tokyo (JP)
(72) Inventor: Wada, Takatsugu, Kanagawa (JP)
(74) Representative: Klunker . Schmitt-Nilson . Hirsch

(56) References cited:
- EP-A2- 0 118 837
- US-A1- 2003 187 356
- US-A1- 2008 021 328
- WANG HAIFENG ET AL: "Silicon oxide colloidal/polymer nanocomposite films", APPLIED PHYSICS LETTERS, AIP, AMERICAN INSTITUTE OF PHYSICS, MELVILLE, NY, US, vol. 85, no. 24, 1 January 2004 (2004-01-01), pages 5998-6000, XP012063836, ISSN: 0003-6951, DOI: DOI:10.1063/1.1836018

## Description

### BACKGROUND OF THE INVENTION

### 1. Field of the Invention

The present invention relates to an ultrasonic transducer, ultrasonic probe and producing method. More particularly, the present invention relates to an ultrasonic transducer of which sensitivity can be raised with a simple structure, and ultrasonic probe and producing method.

### 2. Description Related to the Prior Art

An ultrasonic probe is widely used for medical diagnosis. An ultrasonic transducer is disposed in a distal tip of the ultrasonic probe. The ultrasonic transducer is constituted by backing material, a piezoelectric layer, electrodes, acoustic matching layers, and an acoustic lens. The ultrasonic transducer emits ultrasonic waves to a human body, and receives reflected ultrasonic waves or echo from the human body. An ultrasonic imaging device is supplied with echo information from the ultrasonic transducer, processes the same electrically to produce an ultrasonic image.

It is also possible to produce ultrasonic tomographic images by emission of the ultrasonic waves in a scanned manner. Various methods of producing the ultrasonic tomographic images are known. In a method of mechanical scan, the ultrasonic transducer is mechanically rotated, swung or slid. In a method of electronic scan, a plurality of the ultrasonic transducers are arranged in an array. One or more of the ultrasonic transducers to be driven are changed over by an electronic switch or the like.

It has been very usual to use one piezoelectric layer to transmit and receive the ultrasonic waves. There is another structure in which a first one of the piezoelectric layers transmits the ultrasonic waves and a second one of the piezoelectric layers receives the reflected ultrasonic waves. For the first one, an inorganic piezoelectric ceramic material is suitably used with a relatively high value of an electromechanical coupling coefficient k33 for high power in transmitting the ultrasonic waves. For a second one, a polymeric piezoelectric compound is suitably used with a small value of a transmission coefficient d33 but with a relatively high value of a reception coefficient g33 for good sensitivity for receiving the reflected ultrasonic waves.

In JP-A 6-148154, one of the piezoelectric layers for transmission is disposed on a proximal surface of the acoustic lens, on a side opposite to the human body. The remainder of the piezoelectric layers for reception are disposed in recesses formed in the acoustic lens, on a distal side near to the human body.

There is a large difference in acoustic impedance between the human body and the ultrasonic transducers. One or more acoustic matching layers are formed for change the difference in the acoustic impedance stepwise to reduce a loss of the ultrasonic waves in the propagation. However, JP-A 6-148154 does not suggest consideration of the difference in the acoustic impedance between the human body and the ultrasonic transducers.

The ultrasonic transducers disclosed in JP-A 6-148154 have a problem of a high manufacturing cost due to a complicated structure in which a recess is formed in the acoustic lens and the ultrasonic transducers for reception are disposed in the recess.

US 2003/0187356 A1 discloses an ultrasonic transducer in which an acoustic matching layer constituting an electrode of the piezoelectric layer is provided. With respect to the material of the matching layer, a carbon composite material is mentioned, comprising graphite containing fine particles of SiCB₄C.

### SUMMARY OF THE INVENTION

In view of the foregoing problems, an object of the present invention is to provide an ultrasonic transducer of which sensitivity can be raised with a simple structure, and ultrasonic probe and producing method.

In order to achieve the above and other objects and advantages of this invention, an ultrasonic transducer includes the features of claim 1.

The second piezoelectric layer is positioned nearer to the object than the first piezoelectric layer.

The first piezoelectric layer is inorganic, and the second piezoelectric layer is organic.

The first piezoelectric layer is inorganic, and the second piezoelectric layer is a complex containing an organic compound and an inorganic compound dispersed in the organic compound.

The acoustic matching layer contains metal.

The metal is constituted by silver.

The acoustic matching layer is a complex containing an organic compound and metal.

The organic compound is adhesive.

The metal is constituted by metal nano particles.

The acoustic matching layer includes a first layer of a material with a relatively low conductivity or a non-conductive material. A conductive material is overlaid on the first layer.

Furthermore, a second acoustic matching layer is overlaid on a surface of the second piezoelectric layer opposite to the acoustic matching layer.

Each of the first and second piezoelectric layers includes plural element regions arranged in an array in the first direction.

Preferably, the second piezoelectric layer includes plural receiving element regions, arranged in a first direction, and having volumes different from one another.

The plural element regions have receiving surfaces, which are arranged in the first direction, for receiving the reflected ultrasonic waves, and of which areas are different from one another.

The plural element regions have thicknesses different from one another in a direction perpendicular to receiving surfaces thereof.

The element regions are arranged two-dimensionally in the first direction and a second direction.

Also, an ultrasonic probe having such an ultrasonic transducer is provided.

Consequently, sensitivity of the ultrasonic probe can be raised with a simple structure, because the acoustic matching layer is disposed between the first and second piezoelectric layers.

### BRIEF DESCRIPTION OF THE DRAWINGS

The above objects and advantages of the present invention will become more apparent from the following detailed description when read in connection with the accompanying drawings, in which:
Fig. 1 is a perspective view illustrating an ultrasonic diagnostic apparatus;
Fig. 2 is an explanatory view illustrating a layered structure of an ultrasonic transducer array;
Fig. 3 is a block diagram schematically illustrating circuit elements in the ultrasonic transducer array;
Fig. 4 is an explanatory view illustrating one preferred ultrasonic transducer array in which a receiving piezoelectric layer has receiving element regions;
Fig. 5 is an explanatory view illustrating another preferred ultrasonic transducer array having three acoustic matching layers;
Fig. 6 is a flow chart illustrating a sequence of fabricating the ultrasonic transducer array;
Fig. 7 is an explanatory view illustrating a comparative structure of an ultrasonic transducer array having two acoustic matching layers.

### DETAILED DESCRIPTION OF THE PREFERRED EMBODIMENT(S) OF THE PRESENT INVENTION

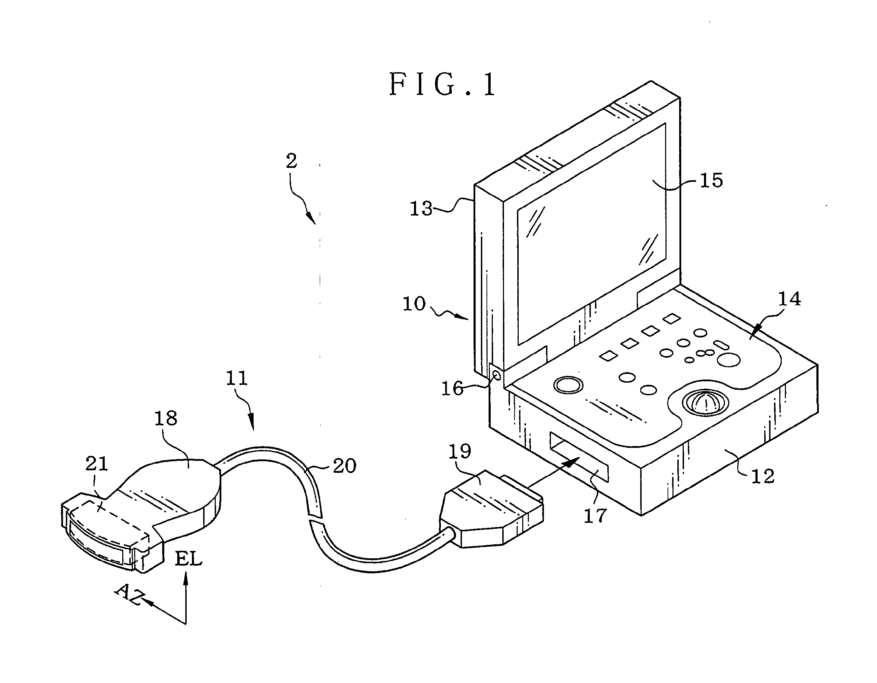

In Fig. 1, an ultrasonic diagnostic system 2 or apparatus includes a portable ultrasonic imaging apparatus 10 and an ultrasonic probe 11. The ultrasonic imaging apparatus 10 or device includes a housing 12 and a cover 13 or display unit. An input panel 14 is disposed on an upper surface of the housing 12, includes plural buttons, trackball and the like, and is operated for inputting various data. A monitor display panel 15 is secured to the inside of the cover 13 for displaying various menus, ultrasonic images, and the like.

A hinge 16 keeps the cover 13 rotatable on the housing 12. The cover 13 is rotatable between an open position and a closed position (not shown), and when in the open position, reveals the input panel 14 and the display panel 15, and when in the closed position, covers and protects the panels 14 and 15 by opposing an inner surface of the cover 13 to an upper surface of the housing 12. A grip (not shown) is secured to a lateral face of the housing 12, and held manually for carrying the ultrasonic imaging apparatus 10 when the cover 13 is in the closed position on the housing 12. A connecting port 17 is formed in another surface of the housing 12 for connecting the ultrasonic probe 11 removably.

The ultrasonic probe 11 includes a scan head 18, a connector plug 19, and a cable 20. The scan head 18 is manually held by a doctor or operator, and caused to touch a patient's body. The cable 20 extends between the scan head 18 and the connector plug 19. An ultrasonic transducer array 21 or UT array is incorporated in an end portion of the scan head 18.

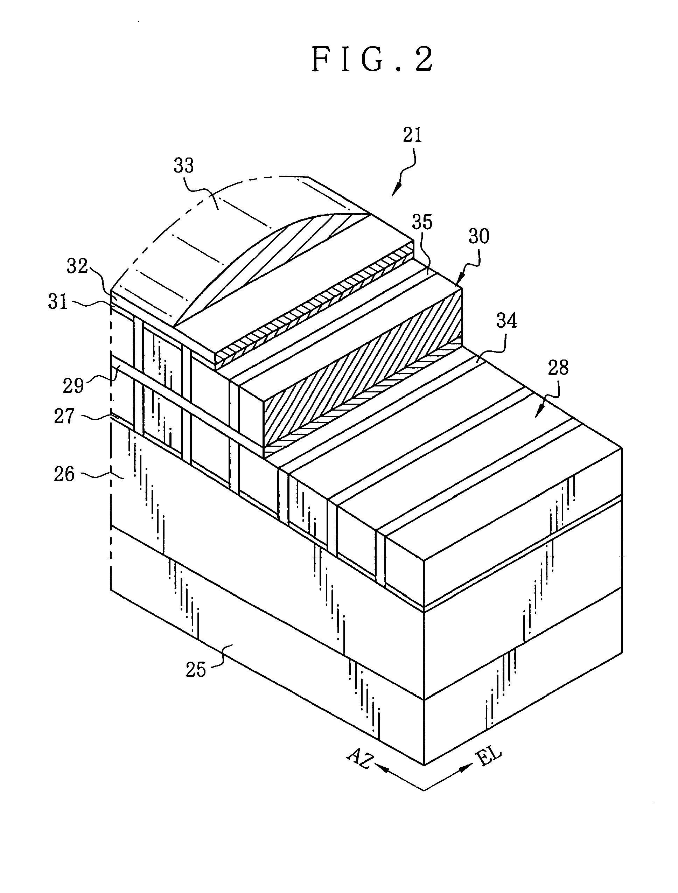

In Fig. 2, the ultrasonic transducer array 21 includes a base plate 25 and various layers overlaid thereon. The base plate 25 is formed from glass, epoxy resin or the like. The layers include backing material 26, a lower electrode 27, a transducer piezoelectric layer 28, a first acoustic matching layer 29, a receiving piezoelectric layer 30, an upper electrode 31, a second acoustic matching layer 32, and an acoustic lens 33 in a listed order.

The backing material 26 regulates free vibration of the transducer piezoelectric layer 28 in the course of emitting ultrasonic waves, to raise a resolving power of the ultrasonic waves in an emission direction. Various materials can be used for the backing material 26 with property to absorb vibration, and can be organic or inorganic. Preferable examples of the materials include epoxy resin and other resins, chlorinated polyethylene rubber, natural rubber, styrene-butadiene rubber (SBR) and other rubbers, because they have low acoustic impedance and can absorb vibration without dropping the sensitivity.

The transducer piezoelectric layer 28 is present in an array of plural element regions of strips, which extend in the EL direction. The element regions of the transducer piezoelectric layer 28 are disposed equidistantly in the array of the AZ direction perpendicular to the EL direction. Filler 34 is charged in gaps or clearances between the element regions of the transducer piezoelectric layer 28 and on their peripheral surface. The array of the element regions are shaped by either surface patterning or cutting (dicing).

The transducer piezoelectric layer 28 has a thickness of 0.1-0.5 mm, and is sandwiched by the first acoustic matching layer 29 and the lower electrode 27. The first acoustic matching layer 29 is electrically conductive, and is used as an upper electrode for the transducer piezoelectric layer 28. The lower electrode 27 is a thin film of metal. Lead wires (not shown) are connected with respectively the first acoustic matching layer 29 and the lower electrode 27. The lower electrode 27, the transducer piezoelectric layer 28 and the first acoustic matching layer 29 are combined to constitute an ultrasonic transducer for transmission and reception.

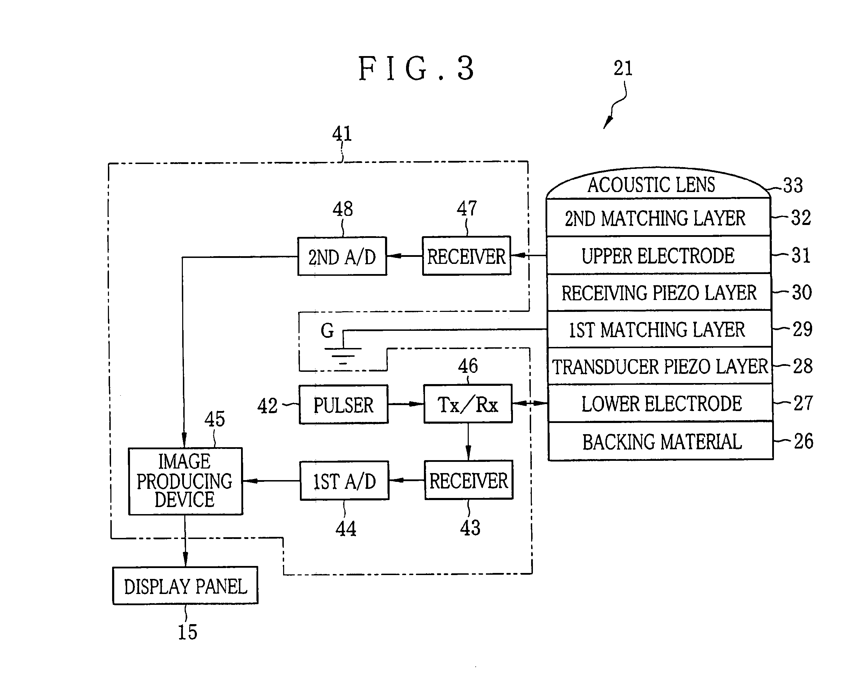

The lead wire connected with the first acoustic matching layer 29 is grounded as illustrated in Fig. 3. A lead wire connected with the lower electrode 27 is connected to the ultrasonic imaging apparatus 10. A transmitter/receiver 41 of Fig. 3 is incorporated in the ultrasonic imaging apparatus 10. When a pulse voltage is applied to the transducer piezoelectric layer 28 by the transmitter/receiver 41, the transducer piezoelectric layer 28 vibrates to generate ultrasonic waves for emission to an object of interest in a body cavity. When the transmitter/receiver 41 receives echo or reflected waves from the object, the transducer piezoelectric layer 28 vibrates to output echo information (reception signal) as a voltage signal.

Various inorganic materials with piezoelectric property can be used for the transducer piezoelectric layer 28. Preferable examples of the materials are PZT and other Pb compounds with piezoelectric property. Among those, specifically preferable examples are PMN-PT and PZN-PT as piezoelectric relaxor single crystal which can have an ultrahigh piezoelectric coefficient. Those are characterized in a high value of the electromechanical coupling coefficient k and in a high value of a ratio of output of ultrasonic waves to applied voltage as efficiency of conversion.

The receiving piezoelectric layer 30 operates also as an acoustic matching layer, and cooperates with the first and second acoustic matching layers 29 and 32. A difference in the acoustic impedance between the transducer piezoelectric layer 28 and a human body is reduced stepwise to increase sensitivity of transmission and reception of ultrasonic waves. The second acoustic matching layer 32 reduces a difference in the acoustic impedance between the receiving piezoelectric layer 30 and the human body stepwise, to increase sensitivity of transmission and reception of ultrasonic waves.

Various conductive materials can be used for the first acoustic matching layer 29 with an acoustic impedance lower than that of the transducer piezoelectric layer 28 and higher than that of the receiving piezoelectric layer 30. A preferable material for the first acoustic matching layer 29 is a baked mixture of metal nano particles (with a diameter of 1-100 nm) and adhesive resin. Preferable metal nano particles are silver nano particles. Specifically, the metal nano particles can exclusively consist of silver nano particles. This is because the silver nano particles have relatively high dispersibility to resin among various metals. When resin with the metal nano particles is baked, particles become bonded in the resin to form a conductive path. Thus, high conductivity can be obtained. Note that the first acoustic matching layer 29 may be a dual layer structure, which can include a first layer of a material without conductivity or a relatively low conductivity, and a conductive layer overlaid on the first layer.

In a manner similar to the transducer piezoelectric layer 28, the receiving piezoelectric layer 30 is constituted by plural element regions of cutting (dicing), or is present with plural element regions of strips of surface patterning for splitting electrically. Filler 35 is charged in gaps of the receiving piezoelectric layer 30 and on its peripheral surface.

The receiving piezoelectric layer 30 has a thickness of 0.05-0.3 mm, and sandwiched by the upper electrode 31 and the first acoustic matching layer 29. The first acoustic matching layer 29 operates as a lower electrode for the receiving piezoelectric layer 30 in addition to operating as upper electrode for the transducer piezoelectric layer 28 as described above. It follows that the first acoustic matching layer 29 functions as a common electrode for the transducer piezoelectric layer 28 and the receiving piezoelectric layer 30. The upper electrode 31 is a film of metal, to which a lead wire (not shown) is connected.

The lead wire connected with the upper electrode 31 is connected to the ultrasonic imaging apparatus 10. When the receiving piezoelectric layer 30 receives echo or reflected waves, echo information is input to the transmitter/receiver 41 in the ultrasonic imaging apparatus 10 in Fig. 3. A combination of the first acoustic matching layer 29, the receiving piezoelectric layer 30 and the upper electrode 31 constitutes an ultrasonic transducer for reception.

Various organic materials with piezoelectric property can be used for the receiving piezoelectric layer 30 with an acoustic impedance lower than that of the first acoustic matching layer 29 and higher than that of the second acoustic matching layer 32. Among those, preferable materials are PVDF, P (VDF-TrFE) and other fluorocarbon polymers. Those are characterized in a high sensitivity factor g, and relatively high sensitivity to ultrasonic waves. Note that the receiving piezoelectric layer 30 can be formed from complex piezoelectric material formed by dispersing an inorganic compound in an organic compound.

Material for the second acoustic matching layer 32 can be a compound of which acoustic impedance is lower than that of the receiving piezoelectric layer 30 and higher than that of a human body. A preferable example of the material is epoxy resin.

The acoustic lens 33 converges ultrasonic waves from the transducer piezoelectric layer 28 at an object of interest in a body cavity. A material for the acoustic lens 33 is silicone rubber with a maximum thickness of 1 mm or so.

Various adhesive materials can be used for adhesion in overlaying plural layers in the ultrasonic transducer array 21. Among those, epoxy resin as adhesive agent is specifically preferable for high acoustic transmittance, high strength in attachment, and low cost.

In Fig. 3, the transmitter/receiver 41 includes a pulser 42, a receiver 43, a first A/D converter 44, an image producing device 45, a transmitting/receiving switch 46 or TX/RX switch, a receiver 47, and a second A/D converter 48.

The pulser 42 is connected with the lower electrode 27 by the transmitting/receiving switch 46. The pulser 42 transmits a pulse voltage or excitation pulse to the lower electrode 27 for the transducer piezoelectric layer 28 to generate ultrasonic waves.

The receiver 43 is connected with the lower electrode 27 by the transmitting/receiving switch 46. The receiver 43 is supplied with echo information (reception signal) from the transducer piezoelectric layer 28 according to reflected waves or echo from the object of interest.

The first A/D converter 44 converts the echo information from the receiver 43 into a digital form according to the A/D conversion. The digital echo data from the first A/D converter 44 is input to the image producing device 45.

The pulser 42 and the receiver 43 are connected with the transmitting/receiving switch 46, which changes over inputs and outputs of those selectively.

The receiver 47 is connected with the upper electrode 31, and amplifies the echo information from the receiving piezoelectric layer 30 in a manner similar to the receiver 43.

The second A/D converter 48 converts the echo information from the receiver 47 into a digital form according to the A/D conversion. The digital echo data from the second A/D converter 48 is input to the image producing device 45. The image producing device 45 produces an ultrasonic image from the echo information input by the first and second A/D converters 44 and 48, and outputs the image to the display panel 15. Various elements included in the transmitter/receiver 41 except for the image producing device 45 are also incorporated for the transducer piezoelectric layer 28 and the receiving piezoelectric layer 30.

Thus, the ultrasonic transducer array can be easily fabricated in a simple structure without high cost, because the first acoustic matching layer 29 with electric conductivity is used as a common electrode for the transducer piezoelectric layer 28 and the receiving piezoelectric layer 30. The first acoustic matching layer 29 can have the high conductivity owing to the use of resin containing silver nano particles, and can be used sufficiently as common electrode.

The resin for the first acoustic matching layer 29 is a thermoset compound and operates as adhesive for the receiving piezoelectric layer 30. The ultrasonic transducer array 21 can be fabricated easily by reducing the number of steps in the fabrication in comparison with a known method in which adhesive agent is applied to attach the receiving piezoelectric layer 30 after forming the first acoustic matching layer. The number of electrodes of a relatively small thickness can be kept small, to prevent drop of the yield of the fabrication.

In the present embodiment, the transducer piezoelectric layer 28 is used for transmission and reception. However, transmission ultrasonic transducer only for transmission without reception can be used instead of the transducer piezoelectric layer 28.

If a transmission line path between the upper electrode 31 and the receiver 47 is very long, a drop of voltage of the echo information between the upper electrode 31 and the receiver 47 will be considerably great according to line resistance of the transmission line path. This will cause degradation of an ultrasonic image according to ultrasonic waves received by the receiving piezoelectric layer 30, to reduce effect of the substance of the receiving piezoelectric layer 30 with high sensitivity.

Thus, the transmission line path for connection between the upper electrode 31 and the receiver 47 should be as short as possible. The receiver 47 is preferably disposed very near to the upper electrode 31. Specifically, the receiver 47 is contained in the scan head 18 instead of containment in the ultrasonic imaging apparatus 10.

### [Example 1]

The ultrasonic transducer array 21 was formed experimentally. The backing material 26 was chlorinated polyethylene rubber and had a thickness of 1 cm. A flexible printed circuit board (FPC) was attached to the backing material 26 by adhesion of epoxy resin of a thermoset type.

A film of the transducer piezoelectric layer 28 was formed from C92H (trade name, manufactured by Fuji Ceramics Corporation) as piezoelectric ceramic PZT material. The film of the transducer piezoelectric layer 28 was polished for both its surfaces to have a thickness of 260 microns. A film of metal was formed on one surface of the transducer piezoelectric layer 28 by sputtering Ti, Pt and Au one after another. Then a proximal side of the transducer piezoelectric layer 28 having the metal film was attached to the flexible printed circuit board (FPC) on the backing material 26 by adhesion. To this end, adhesive agent was used, which contained silver nano particles, and was thermally hardened. Acoustic impedance of the transducer piezoelectric layer 28 was approximately 31 Mrayl. Note that the lower electrode 27 was constituted by the flexible printed circuit board (FPC) on the backing material 26 and the metal film of Ti, Pt and Au on the transducer piezoelectric layer 28.

As the first acoustic matching layer 29, a coating of resin containing silver nano particles (produced by Sumitomo Denki Kogyo Co., Ltd.) was applied to the transducer piezoelectric layer 28 with a thickness of λ/4 (where λ is a wavelength of ultrasonic waves). The first acoustic matching layer 29 was hardened by heating at approximately 180 deg. C. for one (1) hour in the atmosphere. The acoustic impedance of the first acoustic matching layer 29 after heating was approximately 12 Mrayl.

The receiving piezoelectric layer 30 was formed from PVDF having acoustic impedance of 4.5 Mrayl. The receiving piezoelectric layer 30 was molded at a thickness of λ/4. An surface electrode or film of metal was formed entirely on one surface of the receiving piezoelectric layer 30. A second surface of the receiving piezoelectric layer 30 opposite to the surface electrode was attached to the first acoustic matching layer 29. For the attachment, adhesive agent containing silver nano particles was used. The surface electrode on the receiving piezoelectric layer 30 constituted the upper electrode 31.

A film of epoxy adhesive agent with acoustic impedance of 2 Mrayl was polished to have a thickness of λ/4, and was attached to the upper electrode 31 as the second acoustic matching layer 32 by use of epoxy adhesive agent of a thermoset type. Similarly, the acoustic lens 33 was attached to the second acoustic matching layer 32.

For further examples, elements not included in Example 1 will be hereinafter described.

### [Example 2]

The transducer piezoelectric layer 28 was formed from PMN-PT (trade name) as a Pb(Mg,Nb)O₃-PbTiO₃ compound (manufactured by JFE Mineral Corporation) for a piezoelectric relaxor single crystal. The film of the transducer piezoelectric layer 28 was polished for both its surfaces to have a thickness of 240 microns. Acoustic impedance of the transducer piezoelectric layer 28 was approximately 22 Mrayl.

In a manner similar to Example 1, the first acoustic matching layer 29 was formed and then hardened by heating at approximately 160 deg. C. for one (1) hour in the atmosphere. The acoustic impedance of the first acoustic matching layer 29 after heating was approximately 11 Mrayl.

### [Comparative example 1]

Each of both surfaces of the transducer piezoelectric layer 28 was coated with metals of Ti, Pt and Au successively by sputtering to form a metal film. After this, the transducer piezoelectric layer 28 was attached to the flexible printed circuit board (FPC) positioned on the backing material 26. Adhesive agent was used for the attachment, was resin containing silver nano particles. The transducer piezoelectric layer 28 on the flexible printed circuit board (FPC) was hardened by heating at approximately 100 deg. C. for one (1) hour in the atmosphere. The transducer piezoelectric layer 28 had acoustic impedance of approximately 31 Mrayl.

An upper electrode was disposed instead of the first acoustic matching layer 29. A film of metal was formed on the transducer piezoelectric layer 28 and was the upper electrode. An acoustic matching layer was formed instead of the receiving piezoelectric layer 30. Epoxy resin dispersion of zirconia particles was used, had acoustic impedance of 8 Mrayl, was polished to have a thickness of λ/4, and was overlaid on the transducer piezoelectric layer 28 as the acoustic matching layer. Epoxy adhesive agent of a thermoset type was used, and hardened by heating.

A film of epoxy adhesive agent with acoustic impedance of 3 Mrayl was polished to have a thickness of λ/4, and was attached as the second acoustic matching layer 32 by use of the epoxy adhesive agent of the thermoset type.

**Table 1**

| | Example 1 | Example 2 | Comparative example 1 |
|---|---|---|---|
| Transducer piezoelectric layer 28 | piezoelectric ceramic PZT, 31 Mrayl | piezoelectric relaxor single crystal, 22 Mrayl | piezoelectric ceramic PZT, 31 Mrayl |
| First acoustic matching layer 29 | resin containing silver nano particles, 12 Mrayl | resin containing silver nano particles, 11 Mrayl | x |
| Receiving piezoelectric layer 30 | PVDF, 4.5 Mrayl | PVDF, 4.5 Mrayl | epoxy resin dispersion of zirconia particles, 8 Mrayl |
| Second acoustic matching layer 32 | epoxy resin, 2 Mrayl | epoxy resin, 2 Mrayl | epoxy resin, 3 Mrayl |

Table 1 above indicated a relationship between materials for the layers in Examples and Comparative examples, and values of the acoustic impedance (Mrayl). The transducer piezoelectric layer 28 was formed from the piezoelectric relaxor single crystal and had acoustic impedance of 22 Mrayl only according to Example 2, but was formed from the piezoelectric ceramic PZT and had acoustic impedance of 31 Mrayl according to the remaining examples.

The first acoustic matching layer 29 of Examples 1 and 2 was formed from resin containing silver nano particles. The acoustic impedance of the first acoustic matching layer 29 was 12 Mrayl in Example 1, and was 11 Mrayl in Example 2. In contrast, the first acoustic matching layer 29 was not present in Comparative example 1.

The receiving piezoelectric layer 30 of Examples 1 and 2 was formed from PVDF with the acoustic impedance of 4.5 Mrayl. In contrast, the resin layer of Comparative example 1 instead of the receiving piezoelectric layer 30 was formed from epoxy resin dispersion of zirconia particles with the acoustic impedance of 8 Mrayl.

The second acoustic matching layer 32 in any of the examples was formed from the epoxy resin. The acoustic impedance of the second acoustic matching layer 32 was 3 Mrayl in Example 1, and was 2 Mrayl in all the remaining examples.

An experiment was conducted to test sensitivity of reception of the ultrasonic transducer array 21 fabricated according to Examples 1 and 2 and Comparative example 1. An ultrasonic wave comes to have a distorted waveform in the course of propagation, and comes to include higher harmonic components with a frequency an integer times the frequency of the fundamental wave. As a result, higher harmonic components were received with high sensitivity in addition to a fundamental wave in any of Examples 1 and 2 and Comparative example 1.

In Comparative Example 1, in contrast, the fundamental wave was received when an output frequency was equal to or more than a predetermined frequency. However, it was impossible to receive second harmonic waves. Note that it was possible to receive second harmonic waves by lowering the output frequency, but the lowered output frequency was insufficient for the ultrasonic imaging.

Thus, it was possible with a high sensitivity to receive second harmonic waves in addition to the fundamental wave owing to the receiving piezoelectric layer 30 separately in combination with the transducer piezoelectric layer 28.

In the above embodiments, the ultrasonic probe is a type of convex scan for extracorporeal use. However, a ultrasonic probe of the invention may be a type of a radial scan or a type of a mechanical scan for mechanically rotating, swinging or sliding a single ultrasonic transducer. The feature of the invention can be used in a ultrasonic probe insertable in an instrument channel in an electronic endoscope for in-vivo use, an ultrasonic endoscope structured integrally with an electronic endoscope.

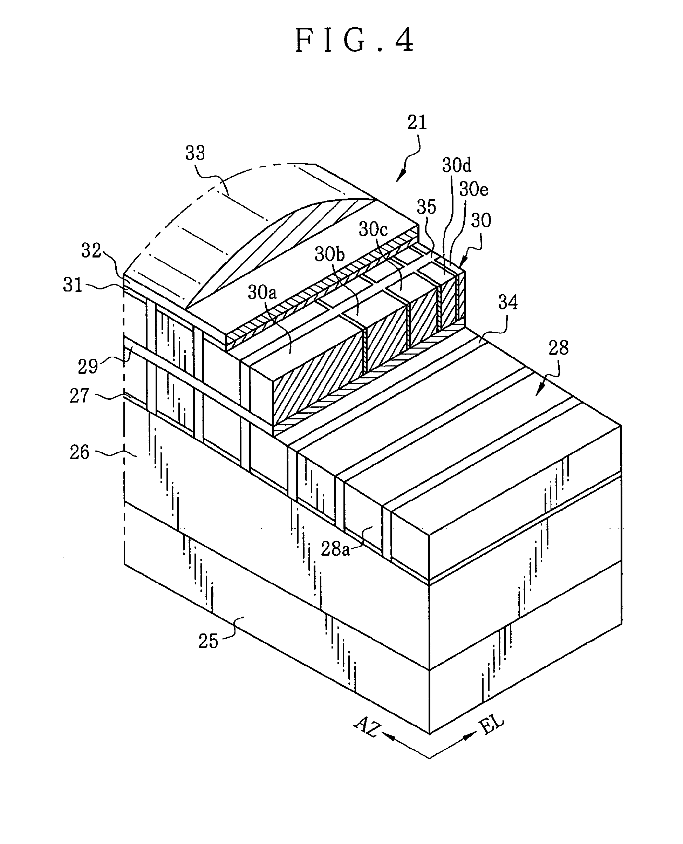

In Fig. 4, another preferred structure of the ultrasonic transducer array 21 is illustrated. The transducer piezoelectric layer 28 in the ultrasonic transducer array 21 includes transducer element regions 28a of the array. The receiving piezoelectric layer 30 includes receiving element regions 30a-30e. The transducer piezoelectric layer 28 and the receiving piezoelectric layer 30 are combined with the base plate 25, the backing material 26, the lower electrode 27, the first acoustic matching layer 29, the upper electrode 31, the second acoustic matching layer 32 and the acoustic lens 33.

The receiving element regions 30a-30e are arranged equidistantly in the AZ direction, and have a two-dimensional multi-element form. The receiving element regions 30a-30e have receiving surfaces for reflected wave with areas decreasing in the EL direction along the receiving piezoelectric layer 30, and have volumes different from one another. Each of the receiving element regions 30a-30e has a specific frequency derived from the volume, shape and the like to receive reflected waves with high sensitivity for specific frequency according to the specific frequency.

Waveforms of the ultrasonic waves become distorted by influence of physical property (hardness and the like) of tissue of a body cavity, and will include harmonic components (non linear components) as an integer times the frequency of the fundamental wave. An area, shape and the like of a receiving surface of the receiving element regions 30a-30e for reflected waves along the receiving piezoelectric layer 30 are predetermined for a specific frequency according to frequencies of the fundamental wave and harmonic waves. This is effective in receiving reflected waves with high sensitivity, especially harmonic components, such as a second harmonic wave, third harmonic wave, fourth harmonic wave and the like.

Also, a non-linear parameter (B/A parameter) can be obtained from a ratio between the received fundamental wave and higher harmonic components as information of possibility of a non linear phenomenon on the tissue in a body cavity. It has been expected to utilize the B/A parameter as diagnostic value of next generation because of correspondence to hardness of the tissue. In the present invention, the B/A parameter can be obtained reliably and easily owing to exact examination.

In the present embodiment, areas of the receiving element regions 30a-30e as receiving surfaces for reflected waves arranged along the receiving piezoelectric layer 30 decreases in the EL direction from each of the two ends toward the center. However, these areas of the receiving element regions 30a-30e can be determined with changes in other manners, for example, can decrease in a direction from the center toward each of the two ends. Also, instead of or in addition to a decrease in the areas of the receiving surfaces of the receiving element regions 30a-30e, it is possible to set the thicknesses different between those in a direction vertical to the receiving surfaces for the reflected waves of the receiving piezoelectric layer 30. When the thickness is reduced to 1/n time as much as the initial thickness (wherein n is an integer), a specific frequency becomes n times as high as before. For example, when the thickness is reduced to a half as much as the initial thickness, the specific frequency becomes two times as high as before.

### [Example 3]

An experiment was conducted for the embodiment. In Example 3, the ultrasonic transducer array 21 of Example 1 was repeated with a difference in using the structure of Fig. 4.

### [Example 4]

Example 2 was repeated with a difference in using the structure of Fig. 4.

### [Comparative example 2]

Comparative example 1 was repeated for comparison.

The experiment was conducted to test sensitivity of reception of the ultrasonic transducer array 21 fabricated according to Examples 3 and 4 and Comparative example 2. As a result, higher harmonic components were received with high sensitivity in addition to a fundamental wave in any of Examples 3 and 4.

In Comparative Example 2, in contrast, the fundamental wave was received when the output frequency was equal to or more than a predetermined frequency. However, it was impossible to receive a higher harmonic component. Note that it was possible to receive a higher harmonic component by lowering the output frequency, but the lowered output frequency was insufficient for the ultrasonic imaging.

Thus, it was possible to receive a higher harmonic component in addition to the fundamental wave owing to the receiving element regions 30a-30e with the different volumes in combination with the transducer element regions 28a.

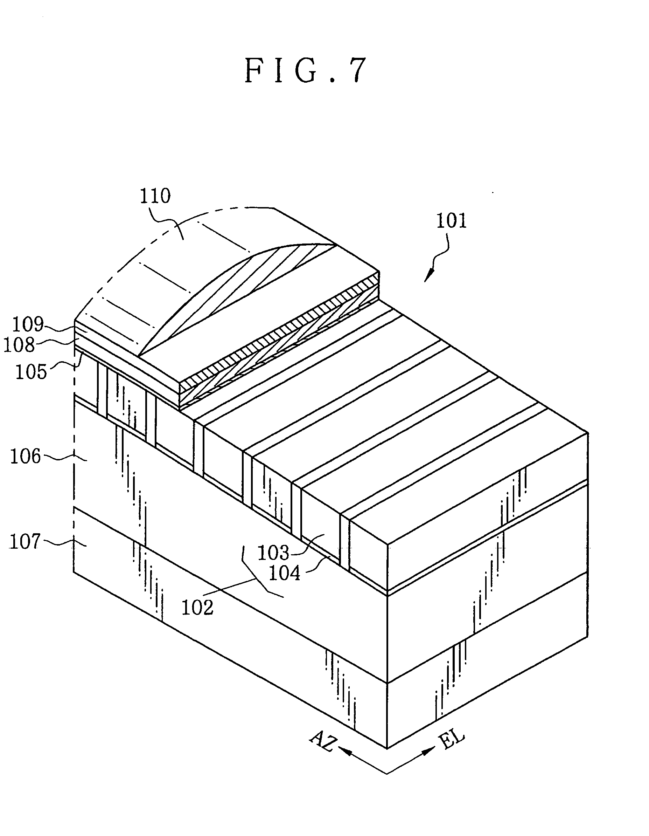

In Fig. 7, a known ultrasonic transducer array 101 or UT array is illustrated in a manner of JP-A 9-139998. Plural ultrasonic transducers 102 of the array are arranged in the AZ direction. The ultrasonic transducers 102 have a piezoelectric layer 103 and electrodes 104 and 105 for applying voltage to the piezoelectric layer 103. Lead wires (not shown) are connected with the electrodes 104 and 105. A base plate 107 and backing material 106 are disposed on a surface of the ultrasonic transducers 102 opposite to an emitting surface for emitting ultrasonic waves. The backing material 106 absorbs unwanted ultrasonic waves from the ultrasonic transducers 102.

There is a considerable difference in the acoustic impedance between the ultrasonic transducers 102 and tissue of a body cavity. That of the tissue is approximately 1.5 Mrayl, in contrast with approximately 25-35 Mrayl as that of piezoelectric ceramic material in the ultrasonic transducers 102. Due to the difference, ultrasonic waves are reflected by an interface between the ultrasonic transducers 102 and the tissue to cause a loss in the propagation. In view of this, a first acoustic matching layer 108 and a second acoustic matching layer 109 are formed on the ultrasonic transducers 102 on their emission surface. That is changed stepwise from the ultrasonic transducers 102 to the tissue. Note that an acoustic lens 110 operates to converge ultrasonic waves in the EL direction which is perpendicular to the AZ direction of the array.

A difference in the acoustic impedance is reduced stepwise by the multi layer structure with plural acoustic matching layers. Theoretically, ultrasonic reflection decreases on an interface of the ultrasonic transducer and tissue of an object of interest, to prevent a loss in the propagation of ultrasonic waves to raise sensitivity in receiving and transmitting ultrasonic waves. However, a total of the interfaces between the acoustic matching layers will be considerably large. Adhesive agent present on the interfaces reflects the ultrasonic waves so as to lower the sensitivity of reception and transmission. Another problem arises in that the plural acoustic matching layers must be attached to one another, so that the yield of the fabrication will be small and the process time will be long due to their small thickness.

If the acoustic matching layer is in the multi layer structure, a first one of acoustic matching layers on an emitting surface of the ultrasonic transducer should satisfy a condition with an impedance which is lower than that of piezoelectric ceramic material and higher than that of organic substances for forming a second one of the acoustic matching layers on the object side (distal side). However, known materials satisfying the condition are very few. A range of selecting the materials is considerably limited.

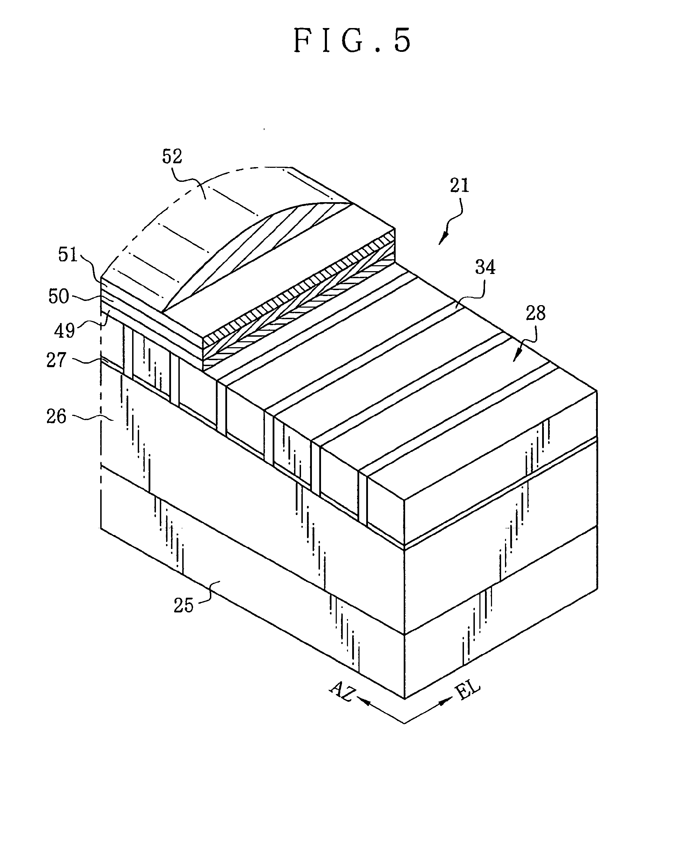

An embodiment to solve the problem is illustrated in Fig. 5. The ultrasonic transducer array 21 includes a first acoustic matching layer 49, a second acoustic matching layer 50, a third acoustic matching layer 51 and an acoustic lens 52 in combination with the base plate 25, the backing material 26, the lower electrode 27 and the transducer piezoelectric layer 28.

Various conductive materials can be used for the first acoustic matching layer 49 with an acoustic impedance lower than that of the transducer piezoelectric layer 28 and higher than that of the second acoustic matching layer 50.

**Table 2**

| | | | |
|---|---|---|---|
| Baking temperature (deg. C.) | 100 | 150 | 200 |
| Sonic speed (m/s) | 1,560 | 1,820 | 2,080 |
| Density (kg/m³) | 3,910 | 5,560 | 7,210 |
| Acoustic impedance (Mrayl) | 6.1 | 10.1 | 15.0 |

Table 2 indicates the sonic speed, density and acoustic impedance of the acoustic matching layer containing silver nano particles as metal nano particles, for each value of the baking temperature. According to the increase in the baking temperature (deg. C.) from 100 and 150 to 200, the sonic speed (m/s) increases in a sequence of 1,560, 1,820 and 2,080. Similarly, the density (kg/m³) increases in a sequence of 3,910, 5,560 and 7,210. The acoustic impedance (Mrayl) increases in a sequence of 6.1, 10.1 and 15.0.

It follows that baking at a high temperature refines the acoustic matching layer to enlarge acoustic impedance. It is possible to determine acoustic impedance at suitable levels by changing the baking temperature in the acoustic matching layer containing silver nano particles. The acoustic matching layer can be used suitably in many types of piezoelectric layers. It is unnecessary to select materials for forming the acoustic matching layer with a desired acoustic impedance, to minimize labor for manual operation.

Various materials can be used for the second acoustic matching layer 50 with an acoustic impedance lower than that of the first acoustic matching layer 49 and higher than that of the third acoustic matching layer 51. Among those, a preferable material is a mixture of zirconia particles and epoxy resin. Various materials can be used for the third acoustic matching layer 51 with an acoustic impedance lower than that of the second acoustic matching layer 50 and higher than that of a human body. Among those, a preferable material is epoxy resin.

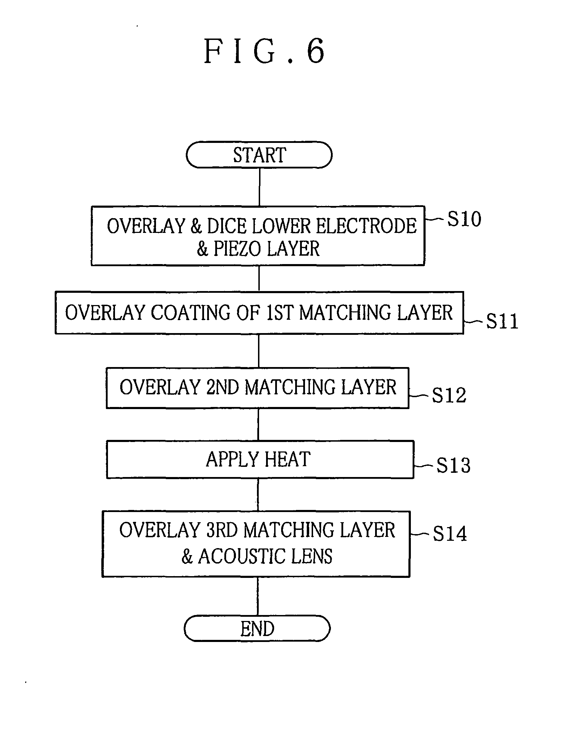

A sequence of fabricating the ultrasonic transducer array 21 is described by referring to Fig. 6. At first, the lower electrode 27 and the transducer piezoelectric layer 28 are overlaid on the backing material 26, and cut in elements of plural strips by dicing in the step S10. The filler 34 is filled in gaps between the elements of the transducer piezoelectric layer 28 and around those. Then an upper surface of the transducer piezoelectric layer 28 is coated with a mixed material as the first acoustic matching layer 49 inclusive of metal nano particles and adhesive resin. See the step S11. Then a mixed material is caused to flow and shaped in a film form, inclusive of zirconia particles and epoxy resin, so then the second acoustic matching layer 50 is overlaid on the first acoustic matching layer 49 of the transducer piezoelectric layer 28. See the step S12. The layers are heated at the step S13. The first acoustic matching layer 49 is hardened by heat to attach the first and second acoustic matching layers 49 and 50 to the transducer piezoelectric layer 28. Also, the third acoustic matching layer 51 and the acoustic lens 52 are overlaid in the step S14 to obtain the ultrasonic transducer array 21. Note that the step of dicing the lower electrode 27 and the transducer piezoelectric layer 28 into the array can be performed at one time for the acoustic matching layers 49-51 after overlaying all the three. In a manner similar to the steps S10-S14, filler is charged in the gaps after the dicing.

As the first acoustic matching layer 49 operates as adhesive to overlay the second acoustic matching layer 50 thereon, the ultrasonic transducer array 21 can be fabricated easily by reducing the number of steps in the fabrication in comparison with a known method in which adhesive agent is applied to form the second acoustic matching layer 50 after forming the first acoustic matching layer. Drop of the yield of the fabrication can be prevented.

In the present embodiment, the acoustic matching layers 49-51 are present. However, the acoustic matching layers can be two or four or more layers which can include one formed from a mixture of metal nano particles and adhesive resin. An increase in the number of the acoustic matching layers can reduce a difference in the acoustic impedance between those. It is possible to reduce a loss in the propagation owing to a decrease in the reflection in the ultrasonic waves on interfaces between the acoustic matching layers.

### [Example 5]

An experiment was conducted for the embodiment. In Example 5, the ultrasonic transducer array 21 of Example 1 was repeated with a difference in using the structure of Fig. 5.

For the first acoustic matching layer 49, the transducer piezoelectric layer 28 was coated with the resin containing silver nano particles at a thickness of λ/4, where λ is a wavelength of ultrasonic waves. For the second acoustic matching layer 50, a film of epoxy resin dispersion of zirconia particles with acoustic impedance of 4 Mrayl was polished to have a thickness of λ/4, and was overlaid on the first acoustic matching layer 49. After this, the second acoustic matching layer 50 was hardened by heating for one (1) hour at approximately 190 deg. C. in the atmosphere. The first acoustic matching layer 49 had acoustic impedance of approximately 14 Mrayl. [Example 6]

Example 2 was repeated with a difference in using the structure of Fig. 5.

### [Comparative example 3]

Comparative example 1 was repeated with the following differences.

The first acoustic matching layer 49 was not present. Instead, a film of metal was formed on the transducer piezoelectric layer 28 and was an upper electrode.

For the second acoustic matching layer 50, epoxy resin dispersion of zirconia particles was used, had acoustic impedance of 8 Mrayl, was polished to have a thickness of λ/4, and was overlaid on the transducer piezoelectric layer 28.

For the third acoustic matching layer 51, a film of epoxy resin was used, had acoustic impedance of 3 Mrayl, was polished to have a thickness of λ/4, and was overlaid on the second acoustic matching layer 50 by use of epoxy adhesive agent of a thermoset type.

### [Comparative example 4]

A film of metal was formed on each of both surfaces of the transducer piezoelectric layer 28 by sputtering Ti, Pt and Au one after another. Then a proximal side of the transducer piezoelectric layer 28 having the metal film was attached to the flexible printed circuit board (FPC) on the backing material 26 by adhesion. To this end, adhesive agent was used, which contained silver nano particles, and was thermally hardened by heating at approximately 100 deg. C. for one (1) hour in the atmosphere. Acoustic impedance of the transducer piezoelectric layer 28 was approximately 31 Mrayl.

In Comparative example 4, an upper electrode was disposed instead of the first acoustic matching layer 49. A film of metal was formed on the transducer piezoelectric layer 28 and was the upper electrode. On the electrode, an acoustic matching layer of glass was overlaid. The acoustic matching layer of glass had acoustic impedance of 14 Mrayl, was polished to have a thickness of λ/4, and was overlaid on the transducer piezoelectric layer 28. Epoxy adhesive agent of a thermoset type was used, and hardened by heating. In general, the acoustic matching layers are diced for shaping as required for the purpose. It was difficult in the acoustic matching layer of glass to dice, due to low working precision according to slipping of a blade for dicing. Also, polishing was very difficult for the acoustic matching layer of glass, to lower the yield of fabrication.

For the second acoustic matching layer 50, epoxy resin dispersion of zirconia particles was used, had acoustic impedance of 4 Mrayl, was polished to have a thickness of λ/4, and was overlaid on an acoustic matching layer of glass. Epoxy adhesive agent of a thermoset type was used, and hardened by heating.

**Table 3**

| | Example 5 | Example 6 | Comparative example 3 | Comparative example 4 |
|---|---|---|---|---|
| Transducer piezoelectric layer 28 | piezoelectric ceramic PZT, 31 Mrayl | piezoelectric relaxor single crystal, 22 Mrayl | piezoelectric ceramic PZT, 31 Mrayl | piezoelectric ceramic PZT, 31 Mrayl |
| First acoustic | resin containing | resin containing | x | glass, 14 Mrayl |
| matching layer 49 | silver nano particles, 14 Mrayl | silver nano particles, 11 Mrayl | | |
| Second acoustic matching layer 50 | epoxy resin dispersion of zirconia particles, 4 Mrayl | epoxy resin dispersion of zirconia particles, 4 Mrayl | epoxy resin dispersion of zirconia particles, 8 Mrayl | epoxy resin dispersion of zirconia particles, 4 Mrayl |
| Third acoustic matching layer 51 | epoxy resin, 2 Mrayl | epoxy resin, 2 Mrayl | epoxy resin, 3 Mrayl | epoxy resin, 2 Mrayl |

Table 3 above indicated a relationship between materials for layers in Examples 5 and 6 and Comparative examples 3 and 4, and values of acoustic impedances (Mrayl).

The first acoustic matching layer 49 of Examples 5 and 6 was formed from resin containing silver nano particles. The acoustic impedance of the first acoustic matching layer 49 was 14 Mrayl in Example 5, and was 11 Mrayl in Example 6. In contrast, the first acoustic matching layer 49 was not present in Comparative example 3. In Comparative example 4, the acoustic matching layer of glass was present instead of the first acoustic matching layer 49, and had acoustic impedance of 14 Mrayl.

The second acoustic matching layer 50 in any of Examples and Comparative examples was formed from epoxy resin dispersion of zirconia particles. The acoustic impedance of the second acoustic matching layer 50 was 8 Mrayl in Comparative example 3, and was 4 Mrayl in the remaining Examples and the remaining Comparative example. The third acoustic matching layer 51 in any of Examples and Comparative examples was formed from epoxy resin. The acoustic impedance of the third acoustic matching layer 51 was 3 Mrayl in Comparative example 3, and was 2 Mrayl in the remaining Examples and the remaining Comparative example.

Results of measurement of the sensitivity (dB) and a fractional bandwidth (%) of the ultrasonic transducer array 21 fabricated according to Examples 5 and 6 and Comparative examples 3 and 4 were indicated in Table 4. The sensitivity was a value representing a ratio of the voltage between the input and output, and was higher according to nearness to zero (0). The fractional bandwidth was a value 100 times a ratio obtained by dividing the band width (Hz) by the center frequency for the sensitivity of -0.6 dB (half of the ratio of the voltage between the input and output). The center frequency was approximately 5 MHz for any of Examples 5 and 6 and Comparative examples 3 and 4.

**Table 4**

| | Sensitivity (dB) | Fractional bandwidth (%) |
|---|---|---|
| Example 5 | -37.7 | 69.2 |
| Example 6 | -35.5 | 70.5 |
| Comparative example 3 | -41.0 | 68.7 |
| Comparative example 4 | -38.6 | 65.3 |

As a result, the sensitivity of Example 5 was still higher than that of Comparative example 3 in spite of nearness in the fractional bandwidth. Note that the number of the acoustic matching layers in Example 5 was two similar to Comparative example 3 on the condition that the first acoustic matching layer 49 was not counted as an acoustic matching layer. Thus, it was possible according to Example 5 to fabricate the ultrasonic transducer array 21 with very high sensitivity even with the same number of steps in the fabrication as Comparative example 3.

It was found in Example 5 that the sensitivity was higher by 1 dB than Comparative example 4, under the condition in that the number of the acoustic matching layers in Example 5 was three similar to Comparative example 4 while the first acoustic matching layer 49 was counted as an acoustic matching layer. This was because an adhesive layer was formed to overlay the acoustic matching layer of glass and the second acoustic matching layer 50, and results in unacceptable effects. In Example 5, however, no adhesive was used to overlay the first and second acoustic matching layers 49 and 50.

It was found in Example 6 that the sensitivity was higher by 2 dB than Example 5. This was because the piezoelectric relaxor single crystal was used with the lower acoustic impedance than the piezoelectric ceramic PZT used in Example 5.

It was possible to construct a multi layer structure for an acoustic matching layer easily owing to the first acoustic matching layer 49 operating also as an upper electrode. Sensitivity was higher than a known transducer in view of the same number of acoustic matching layers.

Although the present invention has been fully described by way of the preferred embodiments thereof with reference to the accompanying drawings, various changes and modifications will be apparent to those having skill in this field. Therefore, unless otherwise these changes and modifications depart from the scope of the present invention defined in the appended claims, they should be construed as included therein.

## Claims

1. An ultrasonic transducer comprising:
a first piezoelectric layer (28) for transmitting ultrasonic waves to an object in a body;
a second piezoelectric layer (30) for receiving said ultrasonic waves reflected by said object; and
an acoustic matching layer (29), disposed between said first and second piezoelectric layers (28, 30), for constituting an electrode common to said first and second piezoelectric layers.

2. An ultrasonic transducer as defined in claim 1, wherein said second piezoelectric layer is positioned nearer to said object than said first piezoelectric layer.

3. An ultrasonic transducer as defined in claim 2, wherein said first piezoelectric layer is inorganic, and said second piezoelectric layer is organic.

4. An ultrasonic transducer as defined in claim 2, wherein said first piezoelectric layer is inorganic, and said second piezoelectric layer is a complex containing an organic compound and an inorganic compound dispersed in said organic compound.

5. An ultrasonic transducer as defined in claim 1, wherein said acoustic matching layer contains metal.

6. An ultrasonic transducer as defined in claim 5, wherein said metal is constituted by silver.

7. An ultrasonic transducer as defined in claim 1, wherein said acoustic matching layer is a complex containing an organic compound and metal.

8. An ultrasonic transducer as defined in claim 7, wherein said organic compound is adhesive.

9. An ultrasonic transducer as defined in claim 7, wherein said metal is constituted by metal nano particles.

10. An ultrasonic transducer as defined in claim 1, wherein said acoustic matching layer includes:
a first layer of a material with a relatively low conductivity or a non-conductive material; and
a conductive material overlaid on said first layer.

11. An ultrasonic transducer as defined in claim 1, further comprising a second acoustic matching layer overlaid on a surface of said second piezoelectric layer opposite to said acoustic matching layer.

12. An ultrasonic transducer as defined in claim 1, wherein each of said first and second piezoelectric layers includes plural element regions arranged in an array in said first direction.

13. An ultrasonic transducer as defined in claim 1, wherein said second piezoelectric layer includes plural receiving element regions, arranged in a first direction, and having volumes different from one another.

14. An ultrasonic transducer as defined in claim 13, wherein said plural element regions have receiving surfaces, which are arranged in said first direction, for receiving said reflected ultrasonic waves, and of which areas are different from one another.

15. An ultrasonic transducer as defined in claim 13, wherein said plural element regions have thicknesses different from one another in a direction perpendicular to receiving surfaces thereof.

16. An ultrasonic transducer as defined in claim 13, wherein said element regions are arranged two-dimensionally in said first direction and a second direction.

17. An ultrasonic probe having an ultrasonic transducer according to any of claims 1 to 16.

18. A method of producing an ultrasonic transducer having a first piezoelectric layer (28), comprising steps of:
placing an acoustic matching layer (29) on said first piezoelectric layer (28), said acoustic matching layer containing a mixture of metal nano particles and adhesive resin,
placing a second piezoelectric layer (30) on said acoustic matching layer (29);
hardening said acoustic matching layer by heating, so as to attach said acoustic matching layer (29) to said piezoelectric layers (28, 30), the acoustic matching layer constituting an electrode common to both piezoelectric layers (28, 30).

## Patentansprüche

1. Ultraschallwandler, umfassend:
eine erste piezoelektrische Schicht (28) zum Senden von Ultraschallwellen zu einem Objekt in einem Körper;
eine zweite piezoelektrische Schicht (30) zum Empfangen der von dem Objekt reflektierten Ultraschallwellen; und
eine akustische Anpassschicht (29), angeordnet zwischen der ersten und der zweiten piezoelektrischen Schicht (28, 30), um eine für die erste und die zweite piezoelektrische Schicht gemeinsame Elektrode zu bilden.

2. Ultraschallwandler nach Anspruch 1, bei dem die zweite piezoelektrische Schicht näher an dem Objekt gelegen ist als die erste piezoelektrische Schicht.

3. Ultraschallwandler nach Anspruch 2, bei dem die erste piezoelektrische Schicht anorganisch und die zweite piezoelektrische Schicht organisch ist.

4. Ultraschallwandler nach Anspruch 2, bei der die erste piezoelektrische Schicht anorganisch ist und die zweite piezoelektrische Schicht ein Komplex ist, der eine organische Verbindung und eine anorganische, in der organischen Verbindung dispergierte Verbindung enthält.

5. Ultraschallwandler nach Anspruch 1, bei dem die akustische Anpassschicht Metall enthält.

6. Ultraschallwandler nach Anspruch 5, bei dem das Metall Silber ist.

7. Ultraschallwandler nach Anspruch 1, bei dem die akustische Anpassschicht ein Komplex ist, der eine organische Verbindung und Metall enthält.

8. Ultraschallwandler nach Anspruch 7, bei dem die organische Verbindung ein Klebstoff ist.

9. Ultraschallwandler nach Anspruch 7, bei dem das Metall durch Metall-Nanopartikel gebildet wird.

10. Ultraschallwandler nach Anspruch 1, bei dem die akustische Anpassschicht enthält:
eine erste Schicht aus einem Werkstoff mit relativ geringer Leitfähigkeit oder einem nicht-leitenden Werkstoff; und
einen leitenden Werkstoff, der über der ersten Schicht liegt.

11. Ultraschallwandler nach Anspruch 1, weiterhin umfassend eine zweite akustische Anpassschicht über einer Oberfläche der zweiten piezoelektrischen Schicht abgewandt von der akustischen Anpassschicht.

12. Ultraschallwandler nach Anspruch 1, bei dem jedes von der ersten und der zweiten piezoelektrischen Schicht mehrere Elementzonen enthält, die in einem Feld in der ersten Richtung angeordnet sind.

13. Ultraschallwandler nach Anspruch 1, bei dem die zweite piezoelektrische Schicht mehrere Empfangselementzonen enthält, die in einer ersten Richtung angeordnet sind und voneinander unterschiedliche Volumina aufweisen.

14. Ultraschallwandler nach Anspruch 13, bei dem die mehreren Elementzonen Empfangsflächen aufweisen, die in der ersten Richtung angeordnet sind, um die reflektierten Ultraschallwellen zu empfangen, und deren Bereiche verschieden voneinander sind.

15. Ultraschallwandler nach Anspruch 13, bei dem die mehreren Elementzonen voneinander verschiedene Dicken in einer Richtung rechtwinklig zu deren Empfangsfläche besitzen.

16. Ultraschallwandler nach Anspruch 13, bei dem die Elementzonen zweidimensional in der ersten und in einer zweiten Richtung angeordnet sind.

17. Ultraschallsonde mit einem Ultraschallwandler nach einem der Ansprüche 1 bis 16.

18. Verfahren zum Fertigen eines Ultraschallwandlers mit einer ersten piezoelektrischen Schicht (28), umfassend folgende Schritte:
Platzieren einer akustischen Anpassschicht (29) auf der ersten piezoelektrischen Schicht (28), wobei die akustische Anpassschicht ein Gemisch aus Metall-Nanopartikeln und einem Klebstoffharz enthält,
Platzieren einer zweiten piezoelektrischen Schicht (30) auf der akustischen Anpassschicht (29);
Aushärten der akustischen Anpassschicht durch Erwärmen, um die akustische Anpassschicht (29) an den piezoelektrischen Schichten (28, 30) zu befestigen, wobei die akustische Anpassschicht eine für beide piezoelektrische Schichten (28, 30) gemeinsame Elektrode bildet.

## Revendications

1. Transducteur à ultrasons comprenant :
une première couche piézoélectrique (28) pour transmettre des ondes ultrasonores en direction d'un objet dans un corps ;
une deuxième couche piézoélectrique (30) pour recevoir lesdites ondes ultrasonores réfléchies par ledit objet ; et
une couche d'appariement acoustique (29), qui est placée entre lesdites première et deuxième couches piézoélectriques (28, 30), pour constituer une électrode commune aux dites première et deuxième couches piézoélectriques.

2. Transducteur à ultrasons selon la revendication 1, dans lequel ladite deuxième couche piézoélectrique est placée plus près dudit objet que ladite première couche piézoélectrique.

3. Transducteur à ultrasons selon la revendication 2, dans lequel ladite première couche piézoélectrique est inorganique et ladite deuxième couche piézoélectrique est organique.

4. Transducteur à ultrasons selon la revendication 2, dans lequel ladite première couche piézoélectrique est inorganique, et ladite deuxième couche piézoélectrique est un complexe contenant un composé organique et un composé inorganique dispersé dans ledit composé organique.

5. Transducteur à ultrasons selon la revendication 1, dans lequel ladite couche de conformation acoustique contient un métal.

6. Transducteur à ultrasons selon la revendication 5, dans lequel ledit métal est constitué par de l'argent.

7. Transducteur à ultrasons selon la revendication 1, dans lequel ladite couche de conformation acoustique est un complexe contenant un composé organique et un métal.

8. Transducteur à ultrasons selon la revendication 7, dans lequel ledit composé organique est une résine adhésive.

9. Transducteur à ultrasons selon la revendication 7, dans lequel ledit métal est constitué par des nanoparticules de métal.

10. Transducteur à ultrasons selon la revendication 1, dans lequel ladite couche de conformation acoustique contient :
une première couche d'un matériau ayant une conductivité relativement faible ou un matériau non conducteur ; et
un matériau conducteur superposé sur ladite première couche.

11. Transducteur à ultrasons selon la revendication 1, comprenant par ailleurs une deuxième couche de conformation acoustique superposée sur une surface de ladite deuxième couche piézoélectrique, à l'opposé de ladite couche de conformation acoustique.

12. Transducteur à ultrasons selon la revendication 1, dans lequel chacune desdites première et deuxième couches piézoélectriques comprend une pluralité de régions d'éléments agencée selon une matrice dans ladite première direction.

13. Transducteur à ultrasons selon la revendication 1, dans lequel ladite deuxième couche piézoélectrique comprend une pluralité de régions d'éléments de réception agencée dans une première direction et ayant des volumes qui sont différents les uns par rapport aux autres.

14. Transducteur à ultrasons selon la revendication 13, dans lequel ladite pluralité de régions d'éléments a des surfaces de réception qui sont agencées dans ladite première direction, pour recevoir lesdites ondes ultrasonores réfléchies, et dont des surfaces sont différentes les unes par rapport aux autres.

15. Transducteur à ultrasons selon la revendication 13, dans lequel ladite pluralité de régions d'éléments a des épaisseurs qui sont différentes les unes par rapport aux autres dans une direction perpendiculaire aux surfaces de réception de ladite pluralité de régions d'éléments.

16. Transducteur à ultrasons selon la revendication 13, dans lequel lesdites régions d'éléments sont agencées en deux dimensions dans ladite première direction et dans une deuxième direction.

17. Sonde à ultrasons comprenant un transducteur à ultrasons selon l'une quelconque des revendications 1 à 16.

18. Procédé de fabrication d'un transducteur à ultrasons ayant une première couche piézoélectrique (28), le procédé comprenant les étapes consistant à :
placer une couche de conformation acoustique (29) sur ladite première couche piézoélectrique (28), ladite couche de conformation acoustique contenant un mélange de nanoparticules de métal et une résine adhésive ;
placer une deuxième couche piézoélectrique (30) sur ladite couche de conformation acoustique (29) ;
faire durcir ladite couche de conformation acoustique en la chauffant, de sorte à fixer ladite couche de conformation acoustique (29) sur lesdites couches piézoélectriques (28, 30), la couche de conformation acoustique constituant une électrode commune aux deux couches piézoélectriques (28, 30).
